Europäisches Patentamt

European Patent Office

Office européen des brevets

(19)

(11) Veröffentlichungsnummer: **0 048 443**

**A1**

(12) **EUROPÄISCHE PATENTANMELDUNG**

(21) Anmeldenummer: **81107327.9**

(22) Anmeldetag: **16.09.81**

(51) Int. Cl.³: **A 61 K 7/48**
**A 61 K 33/00**

(30) Priorität: **17.09.80 DE 3035069**

(43) Veröffentlichungstag der Anmeldung:
**31.03.82 Patentblatt 82/13**

(84) Benannte Vertragsstaaten:
**AT BE CH FR GB IT LI LU NL SE**

(71) Anmelder: **ZYMA GMBH**
**Zielstattstrasse 40**
**D-8000 München 70(DE)**

(72) Erfinder: **Niehaus, Helmut, Dr. med.**
**Radolfzellerstrasse 9a**
**D-8000 München-Pasing(DE)**

(74) Vertreter: **Patentanwälte Grünecker, Dr.Kinkeldey**
**Dr.Stockmair, Dr.Schumann,Jakob, Dr.Bezold Meister,**
**Hilgers, Dr.Meyer-Plath**
**Maximilianstrasse 43**
**D-8000 München 22(DE)**

(54) **Saures dermatologisches Präparat.**

(57) Ein pharmazeutisches Präparat, das Salzsäure enthält und einen pH-Wert von 1,5 oder weniger aufweist, dient als dermatologisches Präparat zur Bekämpfung von Mikroorganismen und ist toxikologisch unbedenklich.

EP 0 048 443 A1

## Beschreibung

Die Erfindung bezieht sich auf ein pharmazeutisches Präparat mit einem Gehalt an einer Halogenwasserstoffsäure.

Bei einem bekannten pharmazeutischen Präparat dieser Gattung (DE-OS 22 38 647) wird als Halogenwasserstoffsäure Fluorwasserstoff verwendet. Die pharmazeutische Wirkung dieses Präparats liegt in erster Linie darin, eine pathologische alkalische Reaktion der Haut zu korrigieren. Da die Fluorwasserstoffsäure keine Sauerstoffverbindungen eingeht und diese Säure somit keinen Sauerstoff übertragen kann, wird weiterhin die Oxidations-Reaktion der Haut erhöht. Aufgrund der kovalenten Bindungen ist die Fluorwasserstoffsäure jedoch in wässriger Lösung kaum dissoziiert und hat dadurch nur einen schwachsauren Charakter. Bei einem pharmazeutischen Präparat mit einem Gehalt an Fluorwasserstoffsäure handelt es sich somit um ein Hautschutzmittel bzw. um ein Mittel, das den normalen physiologischen Säureschutzmantel der Haut wieder herstellt. Wenn die mit dem bekannten Fluorwasserstoffsäurepräparat zu behandelnde Hauterkrankung mikrobielle Ursachen hat, kann keine Wirkung erzielt werden. Die Verwendung von Fluorwasserstoffsäure ist im übrigen physiologisch nicht unbedenklich, da sie den Stoffwechsel der Hautzellen, durch welche sie resorbiert wird, hemmt und dadurch unerwünschte Nebenwirkungen nach sich zieht.

Aus der DE-OS 23 02 216 ist ein Mittel zur Bekämpfung von Hauterkrankungen, Fußpilz, Paradentose, katarrhalischen Erkrankungen der Luftwege sowie von Karzinomen bei Menschen und Bäumen bekannt, das innerlich und äußerlich angewendet werden kann und ursprünglich als

Entroster entwickelt wurde. Es enthält neben Phosphorsäure Eisen, Zink und Mangan in wässriger Lösung.

Der Erfindung liegt die Aufgabe zugrunde, ein pharmazeutisches Präparat bereitzustellen, das eine starke antimikrobielle Wirkung zeigt, den physiologischen Zellstoffwechsel nicht beeinträchtigt und toxikologisch völlig unbedenklich ist.

Diese Aufgabe wird, ausgehend von einem pharmazeutischen Präparat mit einem Gehalt an einer Halogenwasserstoffsäure dadurch gelöst, daß man als Halogenwasserstoffsäure Salzsäure verwendet und daß das Präparat einen pH-Wert von 1,5 oder weniger aufweist.

Infektionen durch Mikroorganismen, wie Pilze, Bakterien oder Viren sind die Ursache vieler schwer zu behandelnder und langwieriger Hauterkrankungen, beispielsweise von Interdigitalmykosen und Nagelmykosen, also Pilzerkrankungen zwischen den Fingern und im Bereich der Nägel, weiterhin von Erythrasma, einer schmetterlingsförmigen Flechte zwischen den Beinen, Pityriasis versicolor usw. Einige dieser Hauterkrankungen erweisen sich bereits als resistent gegen herkömmliche Therapien und gegen die Behandlung mit Antibiotika. Besonders Hauterkrankungen, die durch den Befall mit Pilzen verursacht sind, zeigen sich oft als äußerst therapieresistent und rezidivierend.

Die erfindungsgemäß aufgezeigte Möglichkeit, Mikroorganismen abzutöten, besteht nun darin, den pH-Wert der Umgebung so stark zu erniedrigen, daß der mikrobielle Stoffwechsel irreversibel geschädigt wird, wodurch der Mikroorganismus abgetötet und der gewünschte Heilerfolg erzielt wird. Da bisher davon ausgegangen wurde, daß bei der Anwendung von Mitteln mit einem stark sauren pH-Wert auch das behandelte Gewebe geschädigt würde, sind keine pharmazeutischen Präparate verwendet worden, die einen extrem sauren Charakter aufweisen. Bei der Anwendung des erfindungsgemäßen pharmazeutischen Präparats, das unter Verwendung der Halogenwasserstoffsäure Salzsäure einen pH-Wert von 1,5 oder weniger aufweist, zeigte sich jedoch völlig unerwarteterweise, daß die behandelten Gewebeflächen keinen Schaden erleiden, gleichzeitig jedoch eine hervorragende antimikrobielle Wirkung erzielt wird. Im Gegensatz zu dem Präparat des Standes der Technik stellt das erfindungsgemäße Präparat unter Verwendung von Salzsäure ein, eine körpereigene Substanz enthaltendes Präparat dar, die keinerlei unphysiologische Wirkung nach sich zieht, da Salzsäure in die physiologischen Ionen Wasserstoff und Chlorid dissoziiert. Die unerwünschten Nebeneffekte, die durch die Resorption von Fluor auftreten, werden daher vermieden. Das erfindungsgemäße pharmazeutische Präparat hat sich als toxikologisch vollkommen unbedenklich erwiesen. Daß unerwarteterweise keine Schädigung des behandelten Gewebes durch den stark sauren pH-Wert auftritt, scheint durch die biologische Abpufferung in den Hautzellen bewirkt zu werden.

Eine quantitative antimikrobielle Wirkung bei gleichzeitiger optimaler Verträglichkeit wird erreicht, wenn das Präparat einen pH-Wert von 1,5 oder weniger aufweist. Ein derartig saurer pH-Wert kann nicht mit der nur schwach dissoziierbaren Fluorwasserstoffsäure erreicht werden.

Den stärksten antimikrobiellen Effekt bei immer noch ausgezeichneter Verträglichkeit erreicht man, wenn der pH-Wert zwischen 0,5 und 1 liegt. Es hat sich gezeigt, daß $10^7$ Zellen/ml des Bakterienstammes Escherichia coli bereits nach einer Minute quantitativ abgetötet waren und auch nach längerer Anwendung keinerlei Resistenzen zu beobachten waren. Das erfindungsgemäße Präparat ist somit sogar in vielen Fällen einem Antibiotikum vorzuziehen.

Wird das erfindungsgemäße pharmazeutische Präparat unter Verwendung von Salzsäure und geeigneten Trägersubstanzen als Flüssigpräparat hergestellt, erhält man ein ausgezeichnetes Deodorant für die Behandlung von Follikulitis, von Achselhöhlengeruch und bei Schweißfüßen. In dieser flüssigen Form erweist es sich ebenfalls als hochaktive Substanz gegen Kopfschuppen.

Bei flächigen Erkrankungen von haarfreien Hautteilen ist das erfindungsgemäße Präparat in Salbenform besonders zweckmäßig.

Optimal und besonders vorteilhaft ist das Präparat in Form eines Methylcellulosegels. Ein unter Verwendung eines Methylcellulosegels hergestelltes, halbflüssiges, filmbildendes Präparat erfordert keinen Verband und ist dennoch durch die filmbildende Eigenschaft für eine Dauereinwirkung hervorragend geeignet. Dabei werden auch bei lang anhaltender Einwirkung keinerlei nachteilige Folgen für die behandelten Gewebeflächen beobachtet. Ein durch die Behandlung auftretendes Abschuppen oberster Hautschichten ist nicht schädlich, sondern scheint die Heilwirkung zu fördern, da das Präparat auf diese Weise tieferliegende Krankheitsherde erreicht.

Die Wirksamkeit des erfindungsgemäßen pharmazeutischen Präparats mit dem durch die Verwendung von Salzsäure erreichten, extrem niedrigen pH-Wert, besteht unter anderem in der Breitenwirkung, die so unterschiedliche Mikroorganismen wie Pilze, darunter auch Hefen, Bakterien, deren Sporen sowie Viren erfaßt, und die durch praktisch kein anderes pharmazeutisches Präparat erreicht wird, da diese fast immer auf physiologische Eigenarten des jeweiligen Mikroorganismus (Zellwandbildung bei Bakterien usw.) ausgerichtet sind. Das quantitative Erfassen von Mischkulturen mit den genannten Organismen ist nur möglich, da keiner der genannten Organismen einen pH-Wert von 1,5 oder weniger tolerieren kann. Da gleichzeitig neben der intensiven und breiten antimikrobiellen Wirkung keinerlei toxische oder auch nur unphysiologische Wirkungen für die menschliche Haut auftreten, erweist sich das erfindungsgemäße Präparat als ausgezeichnetes Therapeutikum für praktisch alle durch Mikroorganismen verursachten Hautkrankheiten.

In den nachfolgend geschilderten Beispielen wird die Erfindung näher erläutert.

Beispiel 1

Als Ausgangsbasis wird Methylcellulose verwendet. Mit dieser Substanz wird unter Verwendung von Wasser ein 2 %iges Gel hergestellt. Dieses Gel wird mit konzentrierter HCl (ca. 35 %ig) auf den gewünschten pH-Wert eingestellt.

Es wird ein transparentes Gel mit dem gewünschten pH-Wert erreicht.

## Beispiel 2

Als Ausgangsbasis wird nichtionogenes Polyätherglykol verwendet. Bei einem Gehalt von 20 - 22 % ergibt sich eine Mischung, deren Viskosität durch Variation der Temperatur beim Herstellvorgang von flüssig bis pastös variiert werden kann. Die jeweils hergestellte Mischung wird mit Salzsäure auf den gewünschten pH-Wert eingestellt.

ZYMA GmbH
Zielstattstraße 40, 8000 München 70

Saures dermatologisches Präparat

Patentansprüche

1. Pharmazeutisches Präparat zur äußerlichen Anwendung mit einem Behalt an einer Halogenwasserstoffsäure, d a d u r c h   g e k e n n z e i c h n e t , daß die Halogenwasserstoffsäure Salzsäure ist und daß das Präparat einen pH-Wert von 1,5 oder weniger aufweist.

2. Pharmazeutisches Präparat nach Anspruch 1, dadurch gekennzeichnet, daß der pH-Wert 0,5 bis 1 beträgt.

3. Pharmazeutisches Präparat nach Anspruch 1 oder 2, dadurch gekennzeichnet, daß es flüssig oder eine Salbe ist.

4. Pharmazeutisches Präparat nach Anspruch 1 oder 2, dadurch gekennzeichnet, daß es ein mit einem Methylcelluloseprodukt hergestelltes, filmbildendes Gel ist.

# EUROPÄISCHER RECHERCHENBERICHT

<table>
<tr><td colspan="3">EINSCHLÄGIGE DOKUMENTE</td><td>KLASSIFIKATION DER ANMELDUNG (Int. Cl.³)</td></tr>
<tr><td>Kategorie</td><td colspan="2">Kennzeichnung des Dokuments mit Angabe, soweit erforderlich, der maßgeblichen Teile</td><td>betrifft Anspruch</td></tr>
<tr><td>X</td><td>FR - A - 2 128 187 (R. BERNARD)<br><br>* Anspruch; Seite 1, Zeilen 1-16 *</td><td>1-3</td><td rowspan="3">A 61 K 7/48<br>33/00</td></tr>
<tr><td>X</td><td>CHEMICAL ABSTRACTS, Band 76, Nr. 17, 24. April 1972, Seite 104, Nr. 95308n<br>Columbus, Ohio, U.S.A.<br>S. NAGAO et al.: "Effect of sodium hydroxide and hydrochloric acid on human epidermis. Electronmicroscopic study"<br>& ACTA DERMATO-VENEREOL. 1972, 52(1), 11-23<br><br>* Zusammenfassung *</td><td>1,2</td></tr>
</table>

<table>
<tr><td>X</td><td>US - A - 4 223 018 (J. BELLE)<br><br>* Anspruch; Beispiel *</td><td>1</td><td>RECHERCHIERTE SACHGEBIETE (Int. Cl.³)</td></tr>
<tr><td></td><td>CHEMICAL ABSTRACTS, Band 88, Nr. 18, Mai 1978, Seite 327, Nr. 126363f<br>Columbus, Ohio, U.S.A.<br>& JP - A - 77 148 628 (G. OKABE) 10-12-1977<br><br>* Zusammenfassung *</td><td>1</td><td>A 61 K 7/48<br>7/50<br>9/06<br>33/00</td></tr>
</table>

GB - A - 102 879 (R.H. FOWLER)

* Seite 1, Zeilen 22-29; Anspruch *    1

CHEMICAL ABSTRACTS, Band 38, Nr. 3, 10-02-1944, Spalte 767⁹    ./.

### KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 12-01-1982 | WILLEKENS |

| | EUROPÄISCHER RECHERCHENBERICHT | | Nummer der Anmeldung |

Europäisches Patentamt

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | Columbus, Ohio, U.S.A. E. MEYER et al.: "Germicidal activity of alcohol, HCl and Al K sulfate. Their effect on cutaneous flora" | | |
| | & ARCH. SURGERY 47, 468-477 (1943) | | |
| | * Zusammenfassung. * | 1 | |
| | CHEMICAL ABSTRACTS, Band 46, Nr. 3, 10-02-1952, Spalte 1217d Columbus, Ohio, U.S.A. P.B. PRICE: "Disinfection on the skin" | | RECHERCHIERTE SACHGEBIETE (Int. Cl.³) |
| | & DRUG STANDARDS 19, 161-172 (1951) | | |
| | * Zusammenfassung * | 1 | |
| | CHEMICAL ABSTRACTS, Band 78, Nr. 19, 14. Mai 1973, Seite 87, Nr. 119844d Columbus, Ohio, U.S.A. P. HYBASEK et al.: "Liberation of sorbed water from the skin surface. VIII. Desorption curves of human epidermis (stratum corneum) after treatment with hydrochloric acid" | | |
| | & ACTA UNIV. PALACKI. OLOMUC., FAC. MED. 1972, 62, 97-106 | | |
| | * Zusammenfassung * | 1 | |
| A | THE MERCK INDEX, 8. Auflage, 1968, Merck & Co. Rahway, J.J., U.S.A. Seiten 541,683 ./. | | |

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | * Seite 541, Hydrochloric Acid; Seite 683, Methylcellulose * | 1,4 | |
| | -- | | |
| A | H. JANISTYN: "Handbuch der Kosmetika und Riechstoffe". I. Band: Die kosmetischen Grundstoffe. A. Alfred Hüthig Verlag, 1968 Heidelberg, DE. | | |
| | * Seite 655, Methylcellulose. * | 4 | |
| | -- | | RECHERCHIERTE SACHGEBIETE (Int. Cl.³) |
| A | HAGERS HANDBUCH DER PHARMAZEUTISCHEN PRAXIS, 4. Ausgabe, Springer Verlag, 1977, Band VII B, Seiten 118-119 Berlin, DE. | | |
| | * Seite 118, Anwendung - Seite 119, Zeile 14 * | 4 | |
| | ---- | | |